# EUROPEAN PATENT APPLICATION

(11) **EP 4 062 869 A1**
(43) Date of publication of application: **28.09.2022**
(21) Application number: 20890002.7
(22) Date of filing: 12.11.2020
(51) Int. Cl.: A61F 2/07

(54) **DUAL-LAYER LUMEN STENT**

(30) Priority: 21.11.2019 CN 201911150124
(71) Applicant: Shenzhen Lifetech Endovascular Medical Co., Ltd., Shenzhen, Guangdong 518000 (CN)
(72) Inventor: XIAO, Benhao, Shenzhen, Guangdong 518000 (CN); FANG, Yi, Shenzhen, Guangdong 518000 (CN)
(74) Representative: Prinz & Partner mbB
(86) International application number: PCT/CN2020/128323
(87) International publication number: WO 2021/098578

(57) **Abstract**

A double-layer lumen stent (100) includes a main body lumen stent (10) and an outer-layer skirt stent (20) surrounding an outer wall of the main body lumen stent (10); the outer-layer skirt stent (20) includes a support structure (21) and a cover layer (22) arranged on the support structure (21); one end of the outer-layer skirt stent (20) is connected to the outer wall of the main body lumen stent (10); the other end of the outer-layer skirt stent (20) is an opening structure (24) composed of the support structure (21); and the cover layer (22) is formed into an oblique cut shape at an edge of the opening structure (24). In the double-layer lumen stent (100), when a clinician releases the double-layer lumen stent (100) at a human tissue such as a vessel through a delivery device, constraint from the cover layer (22) to the opening structure (24) of the outer-layer skirt stent (20) can be reduced, and the adhesion between the cover layer (22) at the opening structure (24) on the outer-layer skirt stent (20) and the main body lumen stent (10) is reduced, thereby reducing release resistance to the outer-layer skirt stent (20) during the release process of the double-layer lumen stent (100).

## Description

### Technical Field

The present invention relates to the field of medical devices, and more particularly relates to a double-layer lumen stent.

### Background Art

This section provides only background information related to the present disclosure, which is not necessarily the existing art.

In the field of interventional medicine, stents are widely used to treat or alleviate human luminal stenosis or other lesions in clinical practice. Stents can be classified into vascular stents (aortic stents, peripheral vascular stents, etc.) and non-vascular stents (tracheal stents, esophageal stents, etc.) according to different uses and use environments of the stents. This technology for treating lesions with stents generally uses a compressible stent. When used, the compressible stent is first compressed in a delivery system outside a human body, is then delivered to a specified position through a digital imaging (such as computerized tomography (CT), ultrasound or digital subtraction angiography (DSA)) means, and is finally released at a lesion position in a predetermined manner. The delivery system unfolds the compressible stent using balloon dilatation, or unfolds the compressible stent via the self-expanding nature of the stent. The compressible stent is anchored at a specific position by its own radial force and the contraction of a lumen in the human body, so as to regain the function of a tissue or an organ at the lesion position to achieve the purpose of treating or relieving the pain.

As a compressible stent, a covered stent is the first choice for the treatment of an aortic dissection or an aortic aneurysm. A clinician can release an aortic covered stent via minimally invasive means, and the aortic covered stent has the advantages of providing good treatment effect, and minimizing surgical complications. At present, the aortic covered stent is commonly used to treat aortic vessels with branched vessels. In order to prevent the aortic covered stent from affecting blood supply to the branched vessels while treating the aortic vessels, one or more "chimney stents" are released between the aortic covered stent and a vascular wall of the aortic vessel. A blood flow channel for the branched vessel is formed through the "chimney stent", so as to ensure that the aortic covered stent does not cover the branched vessel while covering a crevasse of an aortic dissection, or isolating an aortic aneurysm cavity. With the development of the covered stent technology, the "chimney stent" is developed from a single-layer structure to a multilayer structure. At present, the multilayer "chimney stent" technology also has the following disadvantages: for the fully covered multilayer "chimney stent", after the multilayer "chimney stent" has been completed, the inner and outer-layer stents in the multilayer "chimney stent may adhere to each other to cause a sticking problem or a buckling deformation phenomenon between inner and outer-layer stents, which can prevent the outer-layer stent from expanding naturally after the multilayer "chimney stent" is released, resulting in poor occlusion effect on a slit between the aortic covered stent and the vascular wall of the aortic vessel.

### Summary of the Invention

Based on the above, it is necessary to provide a double-layer lumen stent.

A double-layer lumen stent includes a main body lumen stent and an outer-layer skirt stent surrounding an outer wall of the main body lumen stent; the outer-layer skirt stent includes a support structure and a cover layer arranged on the support structure; one end of the outer-layer skirt stent is connected to the outer wall of the main body lumen stent; the other end of the outer-layer skirt stent is an opening structure composed of the support structure; and the cover layer is formed into an oblique cut shape at an edge of the opening structure.

In the above-mentioned double-layer lumen stent, when a clinician releases the double-layer lumen stent at a human tissue such as a vessel through a delivery device, since the cover layer of the outer-layer skirt stent is formed into the oblique cut at the edge of the opening structure, constraint from the cover layer to the opening structure of the outer-layer skirt stent can be reduced, and the adhesion between the cover layer at the opening structure of the outer-layer skirt stent and the main body lumen stent is reduced, thereby reducing release resistance to the opening structure on the outer-layer skirt stent during the release process of the double-layer lumen stent and avoiding the phenomenon that the opening structure of the outer-layer skirt stent cannot be completely released because the cover layer is adhered to the main body lumen stent during the process of releasing the double-layer lumen stent.

When the above-mentioned double-layer lumen stent is assembled into a delivery sheath, since the edge of the opening structure has a cover layer that is in an asymmetric oblique cut shape, the cover layer at the edge of the opening structure has a relatively small region on a cross-sectional area of an inlet of the delivery sheath, which is conducive to successfully assembling the double-layer lumen stent into the delivery sheath.

The above-mentioned double-layer lumen stent can be used as a "chimney stent" at a branched vessel on an aortic vessel. When the ordinary "chimney stent" is used, the outer-layer skirt stent with the flush opening structure is flush with a cover edge of a corresponding aortic stent. In the double-layer lumen stent of the present invention, the oblique-cut-shaped cover layer is formed at the edge of the opening structure, so that an anchor length between the cover layer at the edge of the opening structure and the vascular wall can be increased; displacement of the outer-layer skirt stent due to an insufficient anchor region can be prevented; and the outer-layer skirt stent has the oblique-cut-shaped cover layer, which is conducive to enabling blood to flow from the side of the oblique cut.

### Brief Description of the Drawings

By reading the detailed description in the preferred specific implementation modes below, various other advantages and benefits will become clear to those of ordinary skill in the art. The accompanying drawings are only used for the purpose of illustrating the preferred implementation modes, and are not considered to limit the present invention. Furthermore, throughout the drawings, the same reference numerals are used to denote the same components. In the drawings:
FIG. 1 is a schematic structural diagram of a double-layer lumen stent according to one embodiment of the present invention;
FIG. 2 is a schematic structural diagram of an opening structure on an outer-layer skirt stent of a first embodiment of the present invention;
FIG. 3 is a schematic structural diagram showing the anchoring of a double-layer lumen stent of one embodiment of the present invention into a vessel;
FIG. 4 is a schematic structural diagram of an opening structure on an outer-layer skirt stent according to a second embodiment of the present invention;
FIG. 5 is a schematic structural diagram of an opening structure on an outer-layer skirt stent according to a third embodiment of the present invention;
FIG. 6 is a schematic structural diagram of a double-layer lumen stent corresponding to the opening structure shown in FIG. 5;
FIG. 7 is a schematic structural diagram of an opening structure on an outer-layer skirt stent according to a fourth embodiment of the present invention; and
FIG. 8 is a schematic structural diagram of an opening structure on an outer-layer skirt stent according to a fifth embodiment of the present invention.

### Detailed Description of the Invention

In order to facilitate an understanding of the prevent invention, the present invention will be described more comprehensively below with reference to related accompanying drawings. Preferred embodiments of the present invention are illustrated in the drawings. However, the present invention can be embodied in many different forms, and is not limited to the embodiments described herein. On the contrary, the purpose of providing these embodiments is to make the content disclosed by the present invention understood more thoroughly and comprehensively.

It should be noted that when one element is referred to as being "connected" to another element, it can be directly connected to the other element or an intermediate element may exist at the same time. The terms "outer", "inner", "end", "circumferential", "axial", "upper", "side", "proximal", "distal", and similar representations used herein are only for the purpose of illustration.

Unless otherwise defined, all technical and scientific terms used herein are the same as meanings of general understandings of those skilled in the art of the present invention. The terms used in the description of the present invention herein are merely to describe the specific embodiments, not intended to limit the present invention. The term "and/or" used herein includes any and all combinations of one or more related listed items.

In order to enable those skilled in the art to better understand the technical solutions in the present application, the following describes the technical solutions clearly and completely in combination with the embodiments of end structures of different types of stents. Much further, this embodiment is only part of the embodiments in the present application, but not all the embodiments. In addition, in a vessel described, a direction can be defined according to a blood flow direction. In the present invention, it is defined that blood flows from a proximal end to a distal end. A specific embodiment of a double-layer lumen stent 10 is as follows:

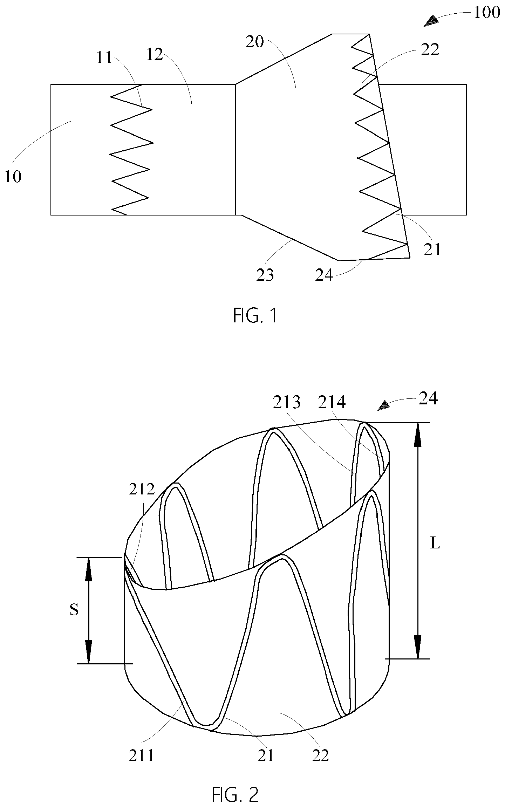

As shown in FIG. 1, the embodiment of the present invention provides a double-layer lumen stent 100. The double-layer lumen stent 100 is composed of a main body lumen stent 10 and an outer-layer skirt stent 20. The main body lumen stent 10 includes an entrance end and an exit end; and the entrance end and the exit end are located in the same axial direction. The outer-layer skirt stent 20 surrounds an outer wall of the main body lumen stent 10. After the double-layer lumen stent 100 is implanted into a vascular lumen, an inner cavity of the main body lumen stent 10 can be used as a blood flow channel. For example, blood can flow into the main body lumen stent 10 through the entrance end of the main body lumen stent 10 and flow out from the exit end of the main body lumen stent 10. Specifically, the main body lumen stent 10 includes at least a support body 11 and a cover structure 12, and the cover structure 12 is attached to an inner wall and an outer wall of the support body 11 to form an inner wall and an outer surface of the main body lumen stent 10.

The outer-layer skirt stent 20 includes a support structure 21 and a cover layer 22 arranged on the support structure 21. In the embodiment illustrated, the outer-layer skirt stent 20 includes a connection structure 23 and an opening structure 24. The connection structure 23 approximates a conical barrel-shaped structure distributed in an axial direction. One end of the connection structure 23 is provided with a connection boundary that is connected to the main body lumen stent 10. The connection boundary on the connection structure 23 and the main body lumen stent 10 may be connected by suturing, twining, or welding. The connection boundary is used for avoiding outward leakage of blood between the main body lumen stent 10 and the outer-layer skirt stent 20. The opening structure 24 is approximately a straight barrel-shaped structure distributed in the axial direction. One end of the opening structure 24 is connected with the other end of the connection structure 23, and the other end of the opening structure 24 is formed into an opening of the outer-layer skirt stent 20. The opening structure 24 is in a closed state during assembly into a delivery sheath and is in an opened state during release in a vessel. It should be noted that in other embodiments, the opening structure 24 may also be a conical barrel-shaped structure. In other words, the opening structure 24 may also be a structure formed by the connection structure 23 that extends away from the connection boundary.

The support structure 21 may be a corrugated metal bar or strip-type metal sheet made of various biocompatible materials, including known materials implanted into a medical device or a combination of various biocompatible materials, such as alloy of 316L stainless steel, cobalt, chromium, nickel, titanium, magnesium and iron, nickel titanium tantalum alloy, and the like, or other biocompatible metal materials. The corrugated metal bar is taken as an example. The support structure 21 may be formed by winding or cutting a metal wire. An internal connection of the support structure 21 may be accomplished by suturing, twining, weaving, cutting, covering, and the like. The cover layer 22 may be an ePTFE film or a PET film. The cover layer 22 may be covered on the inner wall and the outer wall of the support structure 21 via suturing or hot melting and the like.

As shown in FIG. 1 and FIG. 2, according to the first embodiment of the present invention, the cover layer 22 is formed into an oblique cut shape at an edge of the opening structure 24; and an axial length of the cover layer 22 on one side of the opening structure 24 is S, and an axial length of the cover layer 22 on the other side is L. Specifically, the corrugated metal bar at the edge of the opening structure 24 has a plurality of waveform structures distributed in a circumferential direction, and a plurality of axial sizes of the plurality of waveform structures which gradually increase in the circumferential direction. The plurality of waveform structures are formed into oblique cut shapes at the edge of the opening structure 24. The cover layer 22 is covered on the oblique cut shapes of the plurality of waveform structures. In this embodiment, an end opening of the cover layer 22 is flush with an end opening of the support structure 21. In the present application, the waveform structures include two adjacent wave rods and structures connected to the two adjacent wave rods.

Specifically, as shown in FIG. 2, one side of the opening structure 24 is formed into a short wave crest by a first wave rod 211 and a second wave rod 212, and the other side of the opening structure 24 is formed into a high wave crest by a third wave rod 213 and a fourth wave rod 214. The height of the high wave crest is L. The cover layer 22 is covered on the first wave rod 211, the second wave rod 212, the third wave rod 213, and the fourth wave rod 214, and is progressively formed into the oblique cut shape from the short wave crest to the high wave crest. Further, the wave height S is equal to 1/2 to 2/3 of the wave height L, so that the outer-layer skirt stent 20 can be selfexpanded. The wave height design of the short wave crest is approximately the same as the size of a normal wave height. For example, the wave height S of the short wave crest is equal to 1/2 to 2/3 of the normal wave height, so as to enable the short wave crest side of the outer-layer skirt stent 20 to also achieve the effect of occluding a slit between the outer-layer skirt stent 20 and the vascular wall. Even if the short wave crest and the high wave crest are released at the opposite positions during the release process of the outer-layer skirt stent 20, endoleak of the outer-layer skirt stent 20 at the short wave crest due to the wave height can be avoided. In this design, the risk of the endoleak of the outer-layer skirt stent 20 caused by an improper operation can be lowered.

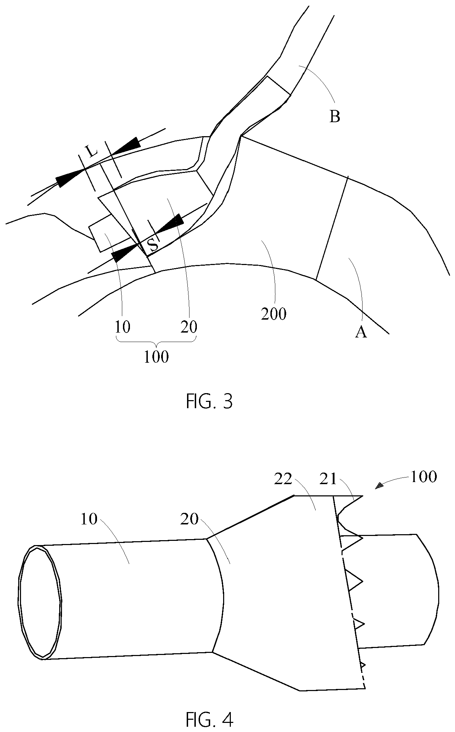

FIG. 3 is a schematic structural diagram showing the anchoring a double-layer lumen stent 100 according to one embodiment of the present invention into a vessel. An aortic vessel A has a branched vessel B. An aortic stent 200 builds a blood flow channel for the aortic vessel A in the aortic vessel A. The double-layer lumen stent 100 serving as a "chimney stent" communicates the aortic stent 200 with the branched vessel B. The main body lumen stent 10 in the double-layer lumen stent 100 extends into the aortic stent 200 to build a blood flow channel for the branched vessel B.

When the clinician releases the double-layer lumen stent 100 at a human tissue such as a vessel through a delivery device, since the cover layer 22 of the outer-layer skirt stent 20 is formed into the oblique cut shape at the edge of the opening structure 24, compared to a cover layer 22 flush in the circumferential direction of the opening structure 24, the oblique-cut-shaped cover layer 22 can reduce constraint to the opening structure 24 on the outer-layer skirt stent 20 and thereby reduce the adhesion between the cover layer 22 at the opening structure 24 on the outer-layer skirt stent 20 and the main body lumen stent 10. This reduces the release resistance to the opening structure 24 on the outer-layer skirt stent 20 during the release process of the double-layer lumen stent 100, thereby avoiding the phenomenon that the opening structure 24 cannot be completely released if the cover layer 22 is adhered to the main body lumen stent 10 during the process of releasing the double-layer lumen stent 100.

Further, when the above-mentioned double-layer lumen stent 100 is assembled into a delivery sheath, since the edge of the opening structure 24 has a cover layer 22 that is in an asymmetric oblique cut shape, the cover layer 22 at the edge of the opening structure 24 has a relatively small region on a cross-sectional area of an inlet of the conveying sheath, which is conducive to successfully assembling the double-layer lumen stent 100 into the delivery sheath. Openings at two ends of the main body lumen stent 10 may also have a cut-shaped structure, which is conducive to smoothly assembling the double-layer lumen stent 100 into the delivery sheath.

Further, the above-mentioned double-layer lumen stent 100 can be used as a "chimney stent" at the branched vessel B on the aortic vessel A. When the ordinary "chimney stent" is used, the outer-layer skirt stent 20 with the flush opening structure 24 is flush with a cover edge of the corresponding aortic stent 200. In the present invention, the oblique-cut-shaped cover layer 22 is formed at the edge of the opening structure 24, so that the short wave crest side of the cover layer 22 is flush with the cover edge of the aortic stent 200, and the high wave crest side of the cover layer 22 is beyond the cover edge of the aortic stent 200, thereby increasing an anchor length between the opening structure 24 and the vascular wall. As a result, displacement of the outer-layer skirt stent 20 due to an insufficient anchor region can be prevented; and the outer-layer skirt stent 20 has the oblique-cut-shaped cover layer 22, which is conducive to enabling blood to flow from the oblique cut. Meanwhile, a transitional region formed by extrusion of the oblique-cut-shaped opening structure 24 on the outer-layer skirt stent 20 and the aortic stent 200 can occlude a gap between them, so as to lower the risk of type-I endoleak.

FIG. 4 is a schematic structural diagram of an opening structure 24 on an outer-layer skirt stent 20 according to a second embodiment of the present invention. A difference from the opening structure 24 in the first embodiment is that the support structure 21 at the edge of the opening structure 24 in the first embodiment has an oblique cut shape, while the support structure 21 at the edge of the opening structure 24 in the second embodiment is a flush structure. The cover layer 22 is formed into an oblique cut shape by means of a gradual decrease in the axial size on the opening structure 24 in the circumferential direction. Specifically, the corrugated metal bar at the edge of the opening structure 24 in the second embodiment has a plurality of waveform structures distributed in the circumferential direction. A plurality of axial sizes of the plurality of waveform structures are the same. The cover layer 22 is covered on the plurality of waveform structures. A plurality of axial sizes of the cover layer 22 on the plurality of waveform structures gradually decreases in the circumferential direction. Specifically, the cover layer 22 extends from a wave trough of the waveform structure of one side to a wave crest of the waveform structure of the other side, so that the cover layer 22 at the edge of the opening structure 24 can be formed into the oblique cut shape according to a linear change. In the double-layer lumen stent 100 adopting the asymmetric cover layer 22 in Embodiment II, the outer-layer skirt stent 20 can self-expand during release.

When the double-layer lumen stent 100 is used as a "chimney stent", it needs to first be located. The main body lumen stent 10 in the double-layer lumen stent 100 extends into a "chimney opening" of other stents using imaging identifiers, so as to build a blood flow channel for a branch vessel. The opening structure 24 on the outer-layer skirt stent 20 extends into an inner wall of the "chimney opening" in a through the use of imaging identifiers. Furthermore, the short edge side of the oblique-cut-shaped cover layer 22 on the outer-layer skirt stent 20 is flush with an edge cover film of other stents. An end position of the oblique-cut-shaped cover layer 22 on the outer-layer skirt stent 20 is beyond the edge cover film of the other stents, so as to occlude the gap and reduce type-I endoleak.

Further, the double-layer lumen stent 100 is anchored through the main body lumen stent 10 and the outer-layer skirt stent 20 after being released in a vessel. A difference from Embodiment I is that an anchor region of the outer-layer skirt stent 20 in Embodiment II is lengthened by L-S. In this way, the risk of displacement of the double-layer lumen stent 100 in the vessel can be lowered, and this is also conducive to reducing type-I endoleak. Meanwhile, compared to a stent with a flush cover layer and a length of L, the double-layer lumen stent 100 in Embodiment II will further lower the difficulty of inserting the double-layer lumen stent 100 into a sheath. In addition, the part of the outer-layer skirt stent 20 beyond the edge cover film of other stents is a bare metal bar. In this way, the phenomenon that the outer-layer skirt stent 20 blocks other branched vessels can be reduced.

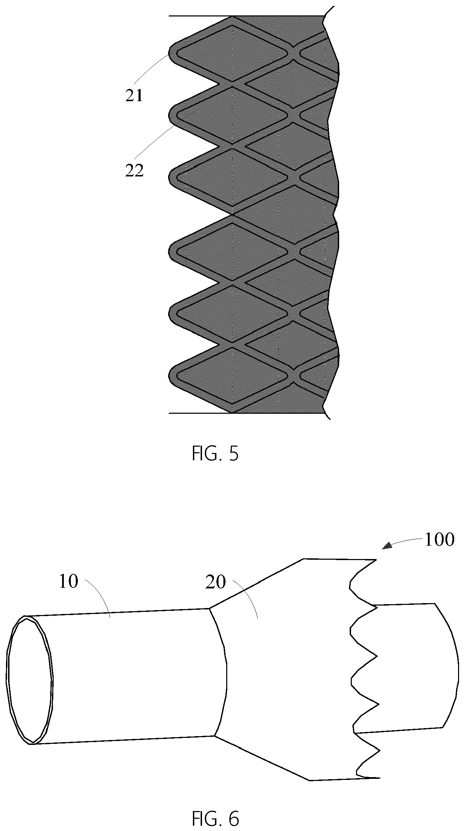

FIG. 5 is a schematic structural diagram of an opening structure 24 on an outer-layer skirt stent 20 according to a third embodiment of the present invention. FIG. 6 is a schematic structural diagram of a double-layer lumen stent 100 corresponding to the opening structure 24 shown in FIG. 5. The corrugated metal bar at the edge of the opening structure 24 has a plurality of waveform structures distributed in the circumferential direction. No cover layer 22 is arranged in the gap between every two adjacent wave crests in the plurality of waveform structures. Specifically, the cover layer 22 uses a petal-shaped cover to cover two adjacent wave crests on the corrugated metal bar, and no cover layer 22 is arranged between two adjacent wave crests, so that the cover layer 22 is formed into a petal structure at the edge of the opening structure 24. The cover layer 22 is made of an ePTFE film or a PET film and is covered on the inner wall and the outer wall of the opening structure 24 via suturing or hot melting and the like.

The petal-shaped cover layer 22 is formed in the edge region of the opening structure 24, so that when the opening structure 24 is assembled into a delivery sheath, part of the edge region of the opening structure 24 is not covered by the cover layer 22, which is conducive to assembling the opening structure 24 into the delivery sheath. When the outer-layer skirt stent 20 is released in the vessel, the region that is not covered by the cover layer 22 can self-expand more easily, so that the entire opening structure 24 can be smoothly expanded during releasing. Meanwhile, when a vascular channel is rebuilt for the double-layer lumen stent 100, blood flow passes through the region of the opening structure 24 that is not covered by the cover layer 22. Further, an odd number of wave crests are designed at the edge of the opening structure 24, which is more conducive to the self-expansion of the opening structure 24. For example, the number of the wave crests at the edge of the opening structure 24 is equal to 2n+5, and n is a natural number. When the number of the waveform structures at the edge of the opening structure 24 is odd, after the double-layer lumen stent 100 is assembled, the phenomenon that two of the plurality of wave crests are adhered together due to an even number of wave crests at the edge of the opening structure 24 is avoided, and the risk that the opening structure 24 cannot expand due to adhesion of the cover layer 22 is lowered.

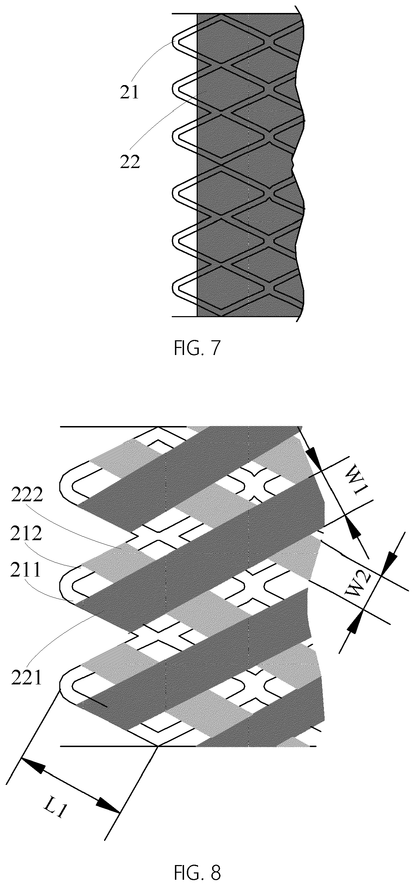

FIG. 7 is a schematic structural diagram of an opening structure 24 on an outer-layer skirt stent 20 according to a fourth embodiment of the present invention. The corrugated metal bar at the edge of the opening structure 24 has a plurality of waveform structures distributed in the circumferential direction. At least part of the plurality of waveform structures close to the edge of the opening structure 24 is not provided with a cover layer 22. Specifically, the wave crest at the edge of the opening structure 24 is composed of two wave rods; only a part of the two wave rods in an axial direction is covered by a cover layer 22. In other words, only half of the wave crest composed of the two wave rods is covered by a cover layer 22, so that a half-bare waveform structure is formed in the edge region of the opening structure 24. When the opening structure 24 is assembled in the conveying sheath, since part of the region of the opening structure 24 is not provided with the cover layer 22, the opening structure 24 is more easily assembled into the delivery sheath. When the opening structure 24 is released, it is easier for the region of the opening structure 24 that is not constrained by the cover layer 22 to self-expand, thereby driving the entire opening structure 24 to smoothly expand. Specifically, an exposed length of the waveform structures at the opening structure 24 is equal to 1/3 to 2/3 of the wave height, which is favorable for insertion into a sheath, and self-expansion of the opening structure 24.

FIG. 8 is a schematic structural diagram of an opening structure 24 on an outer-layer skirt stent 20 according to a fifth embodiment of the present invention.

The cover layer 22 includes a first inclined strip-type cover film 221 arranged at an edge of the opening structure 24. A plurality of first inclined strip-type cover films 221 is distributed at the edge of the opening structure 24 at intervals in the circumferential direction. The cover layer 22 further includes a second inclined strip-type cover film 222 arranged at the edge of the opening structure 24. A plurality of second inclined strip-type cover films 222 is distributed at the edge of the opening structure 24 at intervals in the circumferential direction. The plurality of second inclined strip-type cover films 222 and the plurality of first inclined strip-type cover films 221 cross each other. Specifically, an inner wall of the wave crest region composed of the first wave rod 211 and the second wave rod 212 in the edge region of the opening structure 24 is covered by an intact cover layer, and an outer surface of the wave crest region composed of the first wave rod 211 and the second wave rod 212 is covered by the inclined strip-type cover film. A start end of the first inclined strip-type cover film 221 covers the outer surface of the first wave rod 211, and a start end of the second inclined strip-type cover film 222 covers the outer surface of the second wave rod 212. The first inclined strip-type cover film 221 and the second inclined strip-type cover film 222 both extend in the circumferential direction until the edge of the opening structure 24 is covered by the inclined strip-type cover films in the circumferential direction. The inner wall of the wave crest region composed of the first wave rod 211 and the second wave rod 212 has the intact cover layer, which mainly aims to isolate a lesion region, and the outer wall has the inclined strip-type cover film, which mainly aims to reduce the constraint to the first wave rod 211 and the second wave rod 212. In addition, the inclined strip-type cover films are not limited to being arranged on the inner wall and the outer surface of the opening structure 24. They can also be arranged on the outer surfaces of other parts on the outer-layer skirt stent 20. Specifically, a width of the first inclined strip-type cover film 221 is W1, and a width of the second inclined strip-type cover film 222 is W2, W1=W2=(1/2-2/3)L1; L1 is a length of the first wave rod 211 and the second wave rod 212, so as to facilitate the insertion into a sheath, and self-expansion of the wave rods at the opening structure 24. In addition, the inclined strip-type cover films can achieve higher flexibility of the outer surface of the opening structure 24.

The technical features of the embodiments described above can be arbitrarily combined. In order to make the description concise, all possible combinations of various technical features in the above embodiments are not completely described. However, the combinations of these technical features should be considered as the scope described in this specification as long as there is no contradiction in them.

The above-mentioned embodiments only express several implementation modes of the present invention, and their descriptions are more specific and detailed, but they cannot be understood as limiting the scope of the invention. It should be noted that those of ordinary skill in the art can further make various transformations and improvements without departing from the concept of the disclosure, and these transformations and improvements all fall within the protection scope of the present invention. Therefore, the protection scope of the patent of the present invention shall be subject to the appended claims.

## Claims

1. A double-layer lumen stent, comprising a main body lumen stent and an outer-layer skirt stent surrounding an outer wall of the main body lumen stent, the outer-layer skirt stent comprising a support structure and a cover layer arranged on the support structure, **characterized in that** one end of the outer-layer skirt stent is connected to the outer wall of the main body lumen stent; the other end of the outer-layer skirt stent is an opening structure composed of the support structure; and the cover layer is formed into an oblique cut shape at an edge of the opening structure.

2. The double-layer lumen stent according to claim 1, **characterized in that** the support structure comprises corrugated metal bars distributed in a circumferential direction, and the plurality of corrugated metal bars are distributed in an axial direction to form a barrel-shaped structure.

3. The double-layer lumen stent according to claim 2, **characterized in that** the corrugated metal bar at the edge of the opening structure has a plurality of waveform structures distributed in a circumferential direction, and a plurality of axial sizes of the plurality of waveform structures gradually increase in the circumferential direction; and the plurality of waveform structures are formed into oblique cut shapes at the edge of the opening structure.

4. The double-layer lumen stent according to claim 2, **characterized in that** the corrugated metal bar at the edge of the opening structure has a plurality of waveform structures distributed in a circumferential direction, and a plurality of axial sizes of the plurality of waveform structures are the same; the cover layer is covered on the plurality of waveform structures; and a plurality of axial sizes of the cover layer on the plurality of waveform structures gradually decrease in the circumferential direction.

5. The double-layer lumen stent according to claim 2, **characterized in that** the corrugated metal bar at the edge of the opening structure has a plurality of waveform structures distributed in a circumferential direction, and no cover layer is arranged in a gap between every two adjacent waveforms in the plurality of waveform structures.

6. The double-layer lumen stent according to claim 5, **characterized in that** the number of wave crests of the corrugated metal bar is 2n+5, where n is a natural number.

7. The double-layer lumen stent according to claim 2, **characterized in that** the corrugated metal bar at the edge of the opening structure has a plurality of waveform structures distributed in a circumferential direction, and at least part of the plurality of waveform structures close to the edge of the opening structure is not provided with the cover layer.

8. The double-layer lumen stent according to claim 2, **characterized in that** the cover layer comprises a first inclined strip-type cover film arranged at the edge of the opening structure, and a plurality of first inclined strip-type cover films are distributed at intervals along the edge of the opening structure in the circumferential direction.

9. The double-layer lumen stent according to claim 8, **characterized in that** the cover layer comprises a second inclined strip-type cover film arranged at the edge of the opening structure, and a plurality of second inclined strip-type cover films are distributed at the edge of the opening structure at intervals in the circumferential direction; and the plurality of second inclined strip-type cover films and the plurality of first inclined strip-type cover films cross each other.

10. The double-layer lumen stent according to claim 9, **characterized in that** the corrugated metal bar at the edge of the opening structure has a plurality of waveform structures distributed in a circumferential direction, and two sides of each waveform structure are respectively provided with the first inclined strip-type cover film and the second inclined strip-type cover film.
